# EUROPEAN PATENT APPLICATION

(11) **EP 4 400 052 A1**
(43) Date of publication of application: **17.07.2024**
(21) Application number: 22867351.3
(22) Date of filing: 06.09.2022
(51) Int. Cl.: A61B 5/369, A61B 5/256, A61B 5/291

(54) **BRAIN WAVE MEASUREMENT DEVICE**

(30) Priority: 07.09.2021 JP 2021145735
(71) Applicant: Sumitomo Pharma Co., Ltd., Osaka 541-0045 (JP)
(72) Inventor: YOSHIMIZU, Minoru, Osaka-shi, Osaka 541-0045 (JP); OCHIAI, Yasushi, Tokyo 103-6012 (JP); MASAYOSHI, Sadao, Tokyo 103-0014 (JP)
(74) Representative: Zimmermann & Partner Patentanwälte mbB
(86) International application number: PCT/JP2022/033451
(87) International publication number: WO 2023/038029

(57) **Abstract**

An object of the present invention is to provide a wearable brain wave measurement device having a structure that can achieve both of improvement of durability and improvement of fitting feeling in order to contribute to improvement of the quality of a brain wave signal to be measured. The present invention provides a brain wave measurement device wearable on a living body head, the brain wave measurement device including a housing unit including at least a central front-side component, a left front-side component, a right front-side component, and a rear-side component, the housing unit having a curved shape such that the housing unit is disposed along the living body head from a left temporal region through a frontal region to a right temporal region at a time of wearing, at least one measurement electrode that is fixed to the rear-side component and comes into contact with the frontal region at the time of wearing, and a signal processing unit housed in the housing unit, the signal processing unit processing an electric signal obtained via the measurement electrode. Rigidities of at least two components among the central front-side component, the left front-side component, the right front-side component, and the rear-side component are different from each other.

## Description

### Technical Field

The present invention relates to a brain wave measurement device and, more specifically, to a brain wave measurement device that can measure a brain wave in a state in which the brain wave measurement device is worn on a living body head.

### Background Art

As a wearable brain wave measurement device (a wearable electroencephalograph) worn on the head of a living body such as a human body to measure a brain wave, devices described in Patent Literature 1 (Non-Patent Literature 1) and Non-Patent Literatures 2 and 3 are known. Abrain wave measurement device of Patent Literature 1 (Non-Patent Literature 1) has a double band structure including two electrodes on the frontal region side and configured from an Outer band and an Inner band. Each of the Outer band and the Inner band is an integral component (FIG. 16 of Patent Literature 1). The device of Patent Literature 1 (Non-Patent Literature 1) has a problem in wearing feeling. A brain wave measurement device of Non-Patent Literature 2 is a hairband-type device including two electrodes on the frontal region side. The device has a problem in durability because the device is the hairband type made of a soft material. In the device of Non-Patent Literature 2, a risk of a failure such as wire breakage is considered to be high. A brain wave measurement device of Non-Patent Literature 3 has a structure in which a large number of electrodes project from a main body. Since the device adopts a structure in which the device is worn on a living body head such that the device is disposed from the left temporal region along the occipital region and the right temporal region, it is likely that the electrodes on the frontal region side are not sufficiently fixed. In the device of Non-Patent Literature 3, since a large number of wires from the large number of electrodes project from a main body portion, the device of the Non-Patent Literature 3 has a problem in durability as well.

Most of conventional wearable electroencephalographs are electroencephalographs made of soft materials such as a hairband type electroencephalograph. The electroencephalographs are inferior in durability and have a high frequency of failures such as wire breakages. In contrast, when a wearable electroencephalograph having a housing structure made of a hard material in order to improve durability is used, since the shapes of heads vary depending on persons, it is difficult to manufacture an electroencephalograph that matches the heads of all persons. Therefore, there are, for example, a problem in that a person to be tested (a person who undergoes brain wave measurement) may feel pain in the head by wearing the electroencephalograph and a problem in that the quality of a signal is unstable because, for example, an electrode does not sufficiently come into contact with the head of the person to be tested at the time of wearing or a brain wave is affected by pain felt by the person to be tested.

### Citation List

### Patent Literature

Patent Literature 1: U.S. Patent No. 9867571

### Non-Patent Literature

Non-Patent Literature 1: InteraXon Inc., "MEDITATION REIMAGINED Introducing Muse 2", [online], [searched on August 24, 2021], Internet <URL: https://choosemuse.com/muse-2/>
Non-Patent Literature 2: LAXHAInc., "Bluetooth wireless brain wave meter", [online], [searched on August 24, 2021], Internet <URL: http://www.laxtha.com/ProductView.asp?Model=neuroNicle%20E2>
Non-Patent Literature 3: Emotiv, "EMOTIV EPOC+", [online], [searched on August 24, 2021], Internet <URL: https://www.emotiv.com/epoc/>

### Summary of Invention

### Technical Problem

In view of the above, an object of the present invention is to provide a wearable brain wave measurement device having a structure that can achieve both of improvement of durability and improvement of fitting feeling in order to contribute to improvement of the quality of a brain wave signal to be measured.

### Solution to Problem

In order to solve the problems, the present invention provides a brain wave measurement device wearable on a living body head, the brain wave measurement device including: a housing unit including at least a central front-side component, a left front-side component, a right front-side component, and a rear-side component, the housing unit having a curved shape such that the housing unit is disposed along the living body head from a left temporal region through a frontal region to a right temporal region at a time of wearing; at least one measurement electrode that is fixed to the rear-side component and comes into contact with the frontal region at the time of wearing; and a signal processing unit housed in the housing unit, the signal processing unit processing an electric signal obtained via the measurement electrode, wherein rigidities (stiffness) of at least two components among the central front-side component, the left front-side component, the right front-side component, and the rear-side component are different from each other.

In the brain wave measurement device described above, a number of measurement electrodes may be at least two or more.

In the brain wave measurement device described above, rigidities of the left front-side component and the right front-side component may be higher than rigidity of the central front-side component.

In the brain wave measurement device described above, when the number of measurement electrodes is two or more, centers of respective surfaces of the measurement electrodes coming into contact with the frontal region at the time of wearing may be separated to left and right from each other by 40 mm or more and 90 mm or less along a shape of the rear-side component.

In the brain wave measurement device described above, a contour of a surface of the measurement electrode coming into contact with the frontal region at the time of wearing may have a circular shape having a diameter of 10 mm to 25 mm.

In the brain wave measurement device described above, the signal processing unit may be disposed between the left front-side component and the rear-side component or between the right front-side component and the rear-side component.

### Advantageous Effects of Invention

By using the brain wave measurement device of the present invention, a risk such as wire breakage of a lead wire connecting the electrode and the signal processing unit decreases and durability is improved and, at the same time, wearing feeling of a user (a person to be tested) is improved and the quality of a signal of a measurable brain wave can be improved.

### Brief Description of Drawings

[Figure 1] Figure 1 is a schematic view of (a view from the front oblique downward direction of a user wearing) a brain wave measurement device according to an embodiment of the present invention.
[Figure 2] Figure 2 is a schematic view of (a view from the rear oblique downward direction of the user wearing) the brain wave measurement device according to the embodiment of the present invention.
[Figure 3] Figure 3 is a schematic view of (a view from the upward direction of the user wearing) the brain wave measurement device according to the embodiment of the present invention.
[Figure 4] Figure 4 is a schematic view of (a view from the downward direction of the user wearing) the brain wave measurement device according to the embodiment of the present invention.
[Figure 5] Figure 5 is a schematic view of (a view from the right ear side of the user wearing) the brain wave measurement device according to the embodiment of the present invention.
[Figure 6] Figure 6 is a schematic view of (a view from the left ear side of the user wearing) the brain wave measurement device according to the embodiment of the present invention.
[Figure 7] Figure 7 is a schematic view of the brain wave measurement device according to the embodiment of the present invention (a form in which the brain wave measurement device is worn on the head of the user).
[Figure 8] Figure 8 is a schematic view of a wearing auxiliary band.
[Figure 9] Figure 9 is an exploded view (a perspective view) at the time when a housing unit is disassembled into constituent components.
[Figure 10] Figure 10 is an exploded view (a perspective view) at the time when the housing unit is disassembled into the constituent components.
[Figure 11] Figure 11 is an exploded view (a perspective view) at the time when the housing unit is disassembled into the constituent components.
[Figure 12] Figure 12 is an exploded view (a view from the upward direction of the user wearing the brain wave measurement device) at the time when the housing unit is disassembled into the constituent components.
[Figure 13] Figure 13 is a block diagram showing a configuration of the brain wave measurement device according to the embodiment of the present invention.
[Figure 14] Figure 14 is a block diagram showing a configuration of a data collection terminal device.
[Figure 15] Figure 15 is a flowchart showing operations of the brain wave measurement device according to the embodiment of the present invention and the data collection terminal device.

### Description of Embodiment

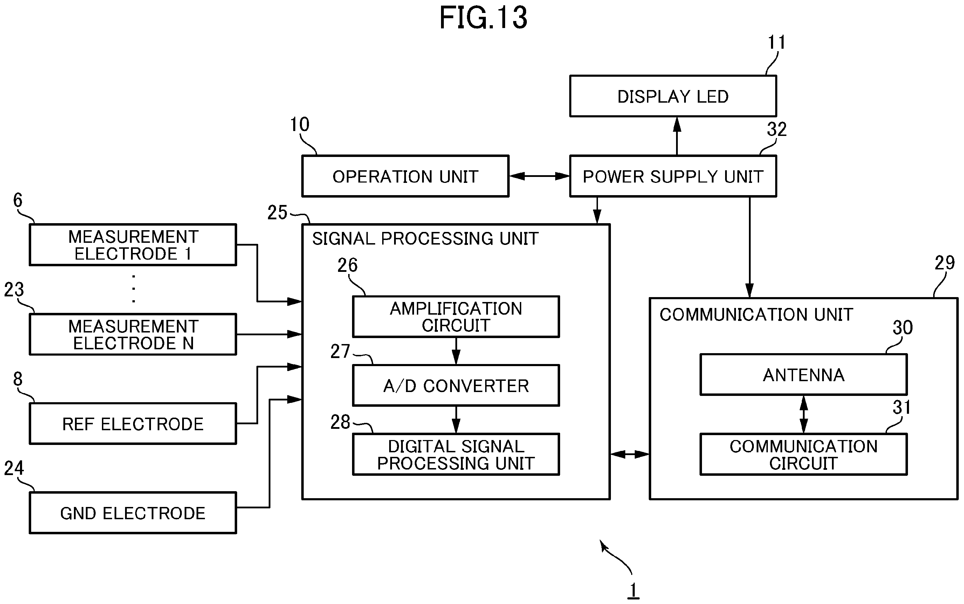

A brain wave measurement device according to an exemplary embodiment of the present invention is described below with reference to the drawings. Note that the brain wave measurement device according to the present invention is not limited to a specific form described below and can be changed as appropriate within the scope of the present invention. Individual functions, elements, and the like included in the embodiment described below can be deleted and changed as appropriate within the scope of the present invention (for example, in Figure 13 referred to below, the brain wave measurement device may be implemented in a form in which a memory device is provided in a signal processing unit and brain wave measurement data is stored in the memory device and a communication unit is not provided) and any functions, elements, and the like not included in the embodiment can be added within the scope of the present invention. For example, in the embodiment described below, the housing unit is described as being configured from four components, that is, a central front-side component, a left front-side component, a right front-side component, and a rear-side component. However, the number of constituent components of the housing unit can be optionally changed (for example, the central front-side component may be disassembled into two components), for example, the number of components can be set to four or more. It is preferable that the materials of the constituent components of the housing unit are insulators. However, at least a part of the constituent components may include a conductive material such as metal in a range in which problems such as short circuit among electrodes and circuit elements do not occur. The constituent components may be made of any material such as silicon, rubber, plastic, or resin, may be made of any plurality of materials, or the individual constituent component can be further disassembled into two or more components. Not only the constituent components of the housing unit but also all constituent elements concerning the brain wave measurement device can be made from any material and can be disassembled into any number of elements unless particularly referred to otherwise. In the embodiment described below, description is made assuming that the number of measurement electrodes is two. However, the number of measurement electrodes can be changed to any number equal to or larger than one, for example, set to one or set to three or more and the positions of the measurement electrodes are also optional. The numbers of reference electrodes and ground electrodes can also be optionally changed (at least one of the reference electrodes and the ground electrodes may not be provided as long as the brain wave measurement device operates). A wearing auxiliary band is not limited to a form described in the embodiment and any band can be used as the wearing auxiliary band. A brain wave measurement device 1 can be implemented even if the wearing auxiliary band is not used. Various functional units described below that perform signal processing and the like can be implemented by any component such as an ASIC (application specific integrated circuit), an embedded system, a microcomputer, or the like. CPUs (central processing units), memory devices, and the like may be included in the various functional units to perform digital information processing. A user in the embodiment described below may be any living thing including a human. A size of the entire brain wave measurement device and sizes of the constituent elements are also optional.

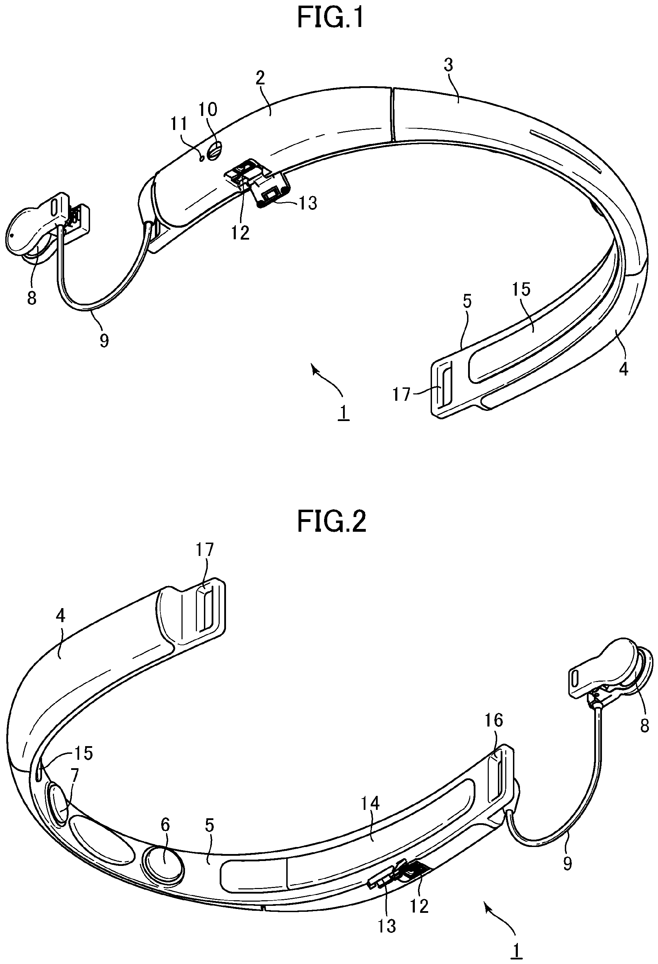

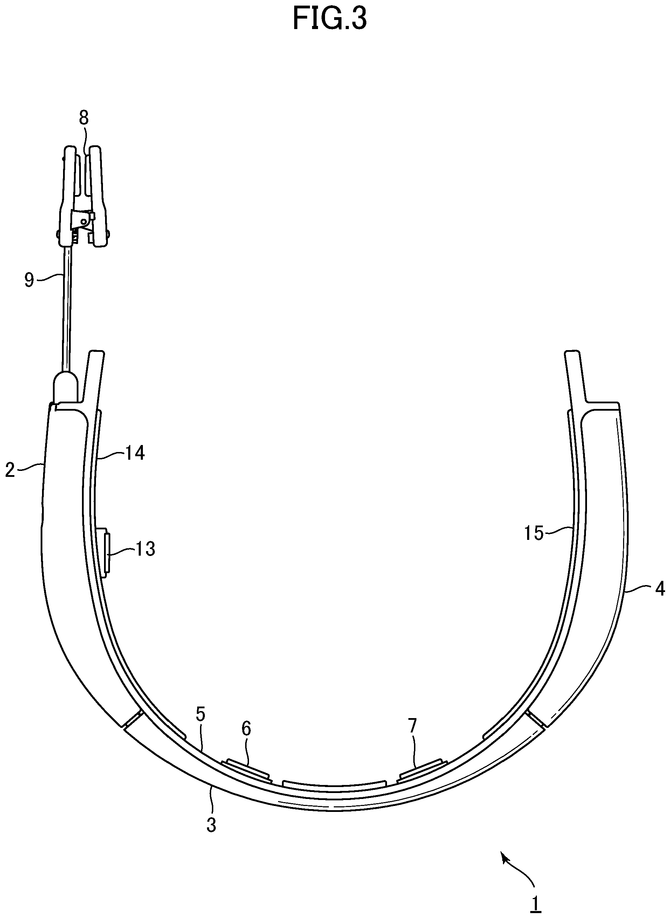

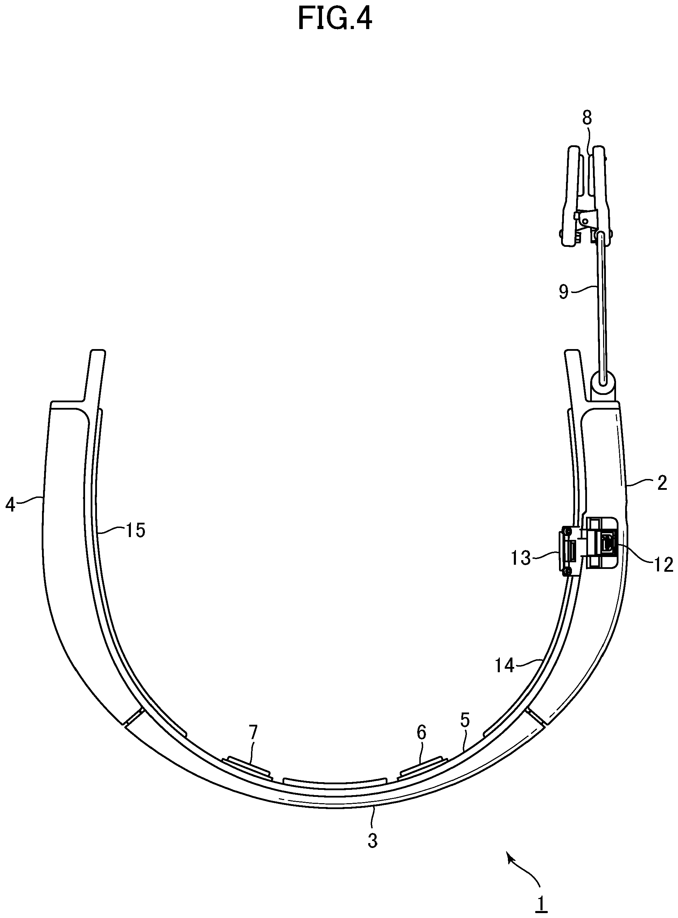

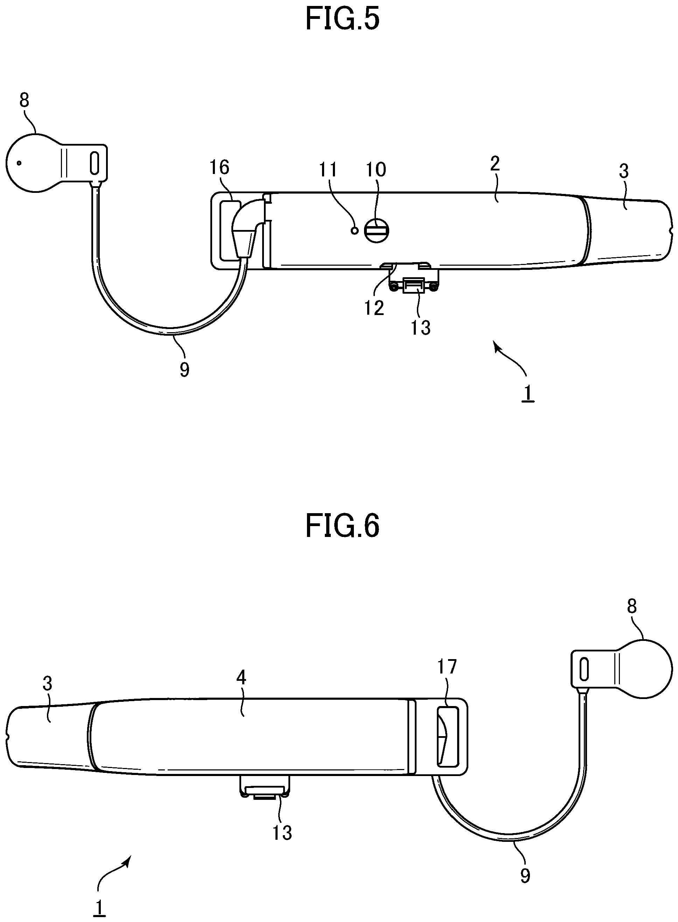

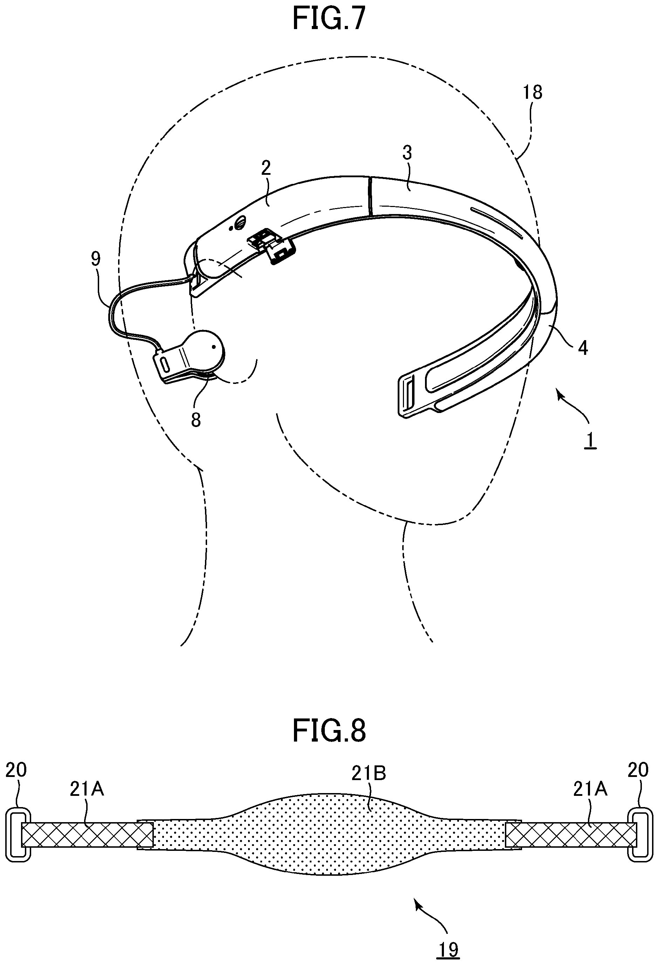

Figure 1 to Figure 6 are schematic views at the times when the brain wave measurement device according to the embodiment of the present invention is viewed respectively from a front oblique downward direction (Figure 1), from a rear oblique downward direction (Figure 2), from the upward direction (Figure 3), from the downward direction (Figure 4), from the right ear side (Figure 5), and from the left ear side (Figure 6) of the user wearing the brain wave measurement device. Figure 7 is a schematic view showing a form in which the brain wave measurement device according to the embodiment of the present invention is worn on the head of the user. As shown in Figure 7, the user wears the brain wave measurement device 1 such that the brain wave measurement device 1 is disposed along the head of the user from the left temporal region to the frontal region and the right temporal region and pinches the ear of the user with a clip-like member (formed from an insulator material) such that reference electrodes 8 disposed on the inner side of the clip-like member (the reference electrodes 8 are each disposed in components on both sides of the clip-like member and two reference electrodes 8 in total are disposed, however, the reference electrodes 8 are collectively referred to as reference electrode 8 in the following description) come into contact with the ear. The size of the brain wave measurement device 1 is optional as described above. However, in one example, the size can be set to height (length in the short side direction at the time when the brain wave measurement device 1 is viewed as shown in Figure 5 and Figure 6) of 30 mm, width (which is length in the left-right direction at the time when the brain wave measurement device 1 is viewed as shown in Figure 3 and Figure 4 and is length in the left-right direction in the user at the time of wearing) of 190 mm, and depth (which is length in the longitudinal direction at the time when the brain wave measurement device 1 is viewed as shown in Figure 3 and Figure 4 and is length in the front-rear direction in the user at the time of wearing) of 150 mm (excluding a protrusion and a leg portion).

When the brain wave measurement device 1 is worn on the head, the housing unit extends in a belt shape toward the left and right auricles along the head. The left and right end portions of the housing unit are each located near the upper parts of the left and right auricles. When the brain wave measurement device 1 is viewed from above, the brain wave measurement device 1 is formed in a semicircular shape, a curvature near the end portions of which is smaller than a curvature in the center thereof. The thickness in the normal direction is the smallest substantially in the center of the housing unit. The width in the up-down direction is also the smallest substantially in the center of the housing unit. The reference electrode 8 may be provided at the end portion on one side in the longitudinal direction of the housing unit.

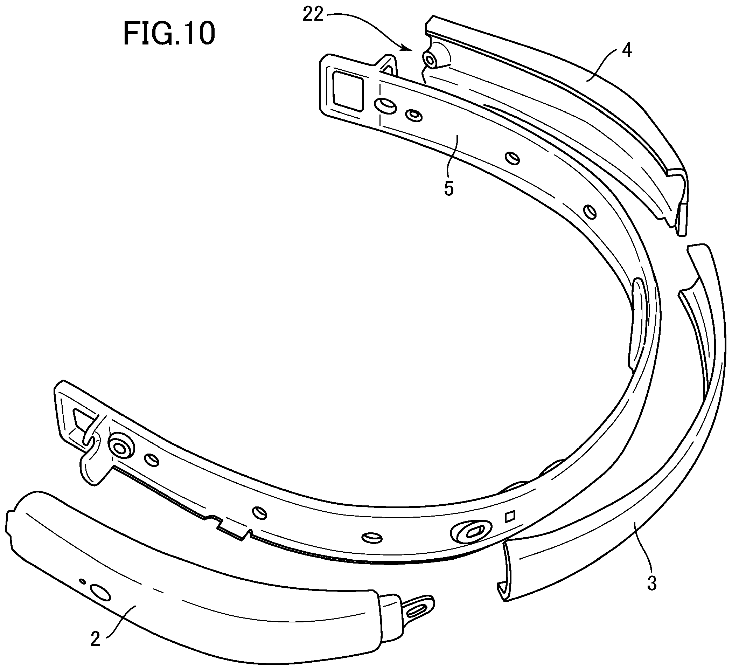

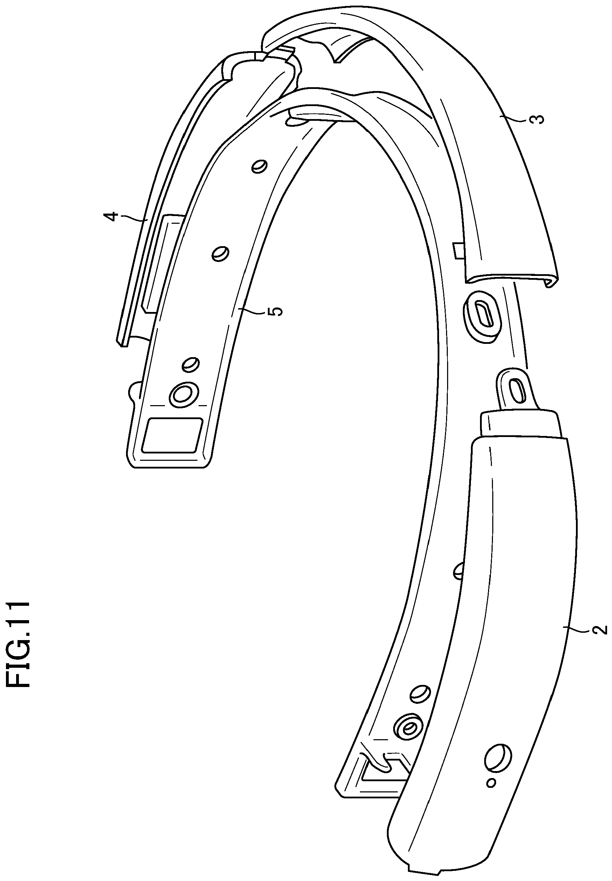

The brain wave measurement device 1 includes a housing unit including a right front-side component 2, a central front-side component 3, a left front-side component 4, and a rear-side component 5, the housing unit having a curved shape such that the housing unit is disposed along the head of the user from a left temporal region through a frontal region to a right temporal region at the time of wearing, at least one measurement electrodes 6 and 7 that are fixed to the rear-side component 5 and come into contact with the frontal region of the user at the time of wearing (in this embodiment, the number of measurement electrodes is set to two, but the number of measurement electrodes may be any number equal to or larger than one as described above), and a signal processing unit (see a signal processing unit 25 in Figure 13 described below) housed in the housing unit, the signal processing unit processing an electric signal obtained via at least one of the measurement electrodes 6 and 7 separated to the left and the right from each other. As described in detail below, the rigidities of at least two components among the central front-side component 3, the left front-side component 4, the right front-side component 2, and the rear-side component 5 are different from each other. In one example, materials and shapes of the components are selected such that the rigidity of the right front-side component 2 and the rigidity of the left front-side component 4 are higher than the rigidity of the central front-side component 3 (more preferably, the rigidity of the right front-side component 2 and the rigidity of the left front-side component 4 are higher than the rigidity of the rear-side component 5). That is, the rigidities of the components can be appropriately set by selecting the materials and the sectional shapes of the components. Note that Figure 1 to Figure 12 are schematic views. The dimensions and the like of the units in the brain wave measurement device 1 are not limited to examples shown in these drawings. However, in one example, the circumferential direction length of the central front-side component 3 may be approximately 30% to approximately 50% of the circumferential direction length (excluding the protrusion and the leg portions; the same applies below) of the brain wave measurement device 1. The circumferential direction length of the left front-side component 4 may be approximately 25 to approximately 35% of the circumferential direction length of the brain wave measurement device 1. The circumferential direction length of the right front-side component 2 may be approximately 25 to approximately 35% of the circumferential direction length of the brain wave measurement device 1. The circumferential direction length of the left front-side component 4 and the circumferential direction length of the right front-side component 2 may be equal to each other or may be different from each other.

The measurement electrode 6 is connected to the signal processing unit 25 via a (coated) lead wire. The measurement electrode 7 is also connected to the signal processing unit 25 via a (coated) lead wire. The brain wave measurement device 1 further includes the reference electrode 8. The reference electrode 8 is connected to the signal processing unit 25 via a (coated) reference electrode lead wire (a lead wire) 9. Besides, when a ground electrode (GND electrode) is provided in the brain wave measurement device 1 (in Figure 13 referred to below, a GND electrode 24. Reference potential in an operation of the brain wave measurement device 1 can be applied by bringing the ground electrode into contact with the head of the user or any position of the body of the user and the reference potential can be used as a reference of potential of the other electrodes), the ground electrode is connected to the signal processing unit 25 via a (coated) lead wire. Note that these electrodes are separately connected to the signal processing unit 25 and configured to input electric signals to the signal processing unit 25 (the (coated) lead wires separately extend from the electrodes and are connected to separate terminals of the signal processing unit 25. Note that wire breakage risks can be reduced by wiring the (coated) lead wires such that the (coated) lead wires other than the reference electrode lead wire 9 pass only the inside of the housing unit (for example, in some cases, a configuration is adopted in which the ground electrode 24 comes into contact with other than the head, and it is not essential to wire the leads in the housing unit)) and the electrodes are not in a short-circuit state. The materials of the electrodes are optional. However, in one example, stainless steel, silver-silver chloride (Ag/AgCl), or silver can be used as the electrode material.

The shape of the measurement electrode 6 and the measurement electrode 7 is optional. However, in one example, contours of surfaces coming into contact with the frontal region of the user at the time of wearing (see Figure 2) are formed to have a circular shape having a diameter of 10 mm to 25 mm (the same applies when three or more measurement electrodes are provided. Adjustment may be made as appropriate to, for example, provide a recess in a part of the circular shape). The shape of the surfaces of the measurement electrode 6 and the measurement electrode 7 coming into contact with the frontal region (the forehead) of the user at the time of wearing is also optional. For example, the shape may be set as a plane as shown in Figure 3 and Figure 4, may be set as a recessed surface (a surface at least partially recessed on the surface coming contact with the frontal region when viewed from the user at the time of wearing), or may be set as a convex surface (a surface at least partially projected on the surface coming into contact with the frontal region when viewed from the user at the time of wearing). However, it is preferable set the shape to a shape other than the convex surface in order to give satisfactory wearing feeling to the user (the same applies when three or more measurement electrodes are provided). By forming the measurement electrode 6 and the measurement electrode 7 in a shape conforming to the frontal region of the user, a contact area increases and brain wave measurement accuracy can be improved. The shapes and sizes of the reference electrode 8 and the ground electrode 24 may be the same and are optional. It is preferable to dispose the measurement electrodes 6 and 7 such that the respective centers of the surfaces of the measurement electrodes 6 and 7 coming into contact with the frontal region at the time of wearing (see Figure 2) are separated to the left and the right from each other by 40 mm or more and 90 mm or less along the shape of the rear-side component 5 (along a curve drawn by the rear-side component 5 when the brain wave measurement device 1 is viewed from the direction shown in Figure 3 and Figure 4) (when three or more measurement electrodes are provided, the measurement electrodes may be disposed at the same interval). This is because it is preferable to set the interval between the measurement electrodes 6 and 7 to approximately 20% of a frontal temporal circumference (length further on the front side than the center of the ears in a head circumference) (further, the user preferably wears the brain wave measurement device 1 such that an intermediate point of respective center points of the surfaces of the measurement electrodes 6 and 7 coming into contact with the frontal region at the time of wearing is located in the center of the frontal region, that is, a position on (an extended line of) the nose ridge line of the user) and, as a result of measuring frontal temporal circumferences of a plurality of humans, the length of 20% of the frontal temporal circumference is considered to be generally fit within a range of 40 mm to 90 mm. For example, an example of the positions of the measurement electrode 6 and the measurement electrode 7 may be the positions of Fp 1 and Fp2 of the international 10-20 system.

A power button 10 is provided as an operation unit in the right front-side component 2. The user presses the power button 10, whereby ON (an operation state) and OFF (a stop state) of the operation of the brain wave measurement device 1 are switched. A display LED (light emitting diode) 11 is provided in the right front-side component 2. Lighting, extinction, flashing, and a light emission color are switched according to the operation state and a charging state. A charging port (a charging inlet) 12 is also provided in the right front-side component 2. A lithium ion battery of a power supply unit 32 (see Figure 13) can be charged by opening a lid 13 of the charging port and connecting a charging cable to the charging port 12. In the rear-side component 5, a (right-side) nonslip sheet 14 is provided in a position corresponding to the right front-side component 2 and a (left-side) nonslip sheet 15 is provided in a position corresponding to the left front-side component 4 to prevent the brain wave measurement device 1 from deviating from the head of the user in a state in which the brain wave measurement device 1 is worn on the head. A material of the nonslip sheets 14 and 15 are optional. However, in one example, urethane, silicon, and the like can be used as the material of the nonslip sheets 14 and 15. A (right-side) auxiliary band attachment hole 16 is provided at the end portion on the right front-side component 2 side in the rear-side component 5 and a (left-side) auxiliary band attachment hole 17 is provided at the end portion on the left front-side component 4 side in the rear-side component 5. One end and the other end of a wearing auxiliary band (belt) 19 shown in Figure 8 are respectively inserted through the auxiliary band attachment holes 16 and 17 to connect the wearing auxiliary band 19 to the brain wave measurement device 1 (in the wearing auxiliary band 19 shown in Figure 8, a ring-like member 20 having a rectangular ring shape attached to a hook-and-loop fastener hook section 21A side at one end is directed sideways (hook-and-loop fastener hook sections 21A and hook-and-loop fastener loop sections 21B on both end sides can be bent), the ring-like member 20 is inserted through the auxiliary band attachment hole 16, the ring-like member 20 on the hook-and-loop fastener hook section 21A side at the other end is directed sideways and the ring-like member 20 is inserted through the auxiliary band attachment hole 17, and the respective hook-and-loop fastener hook sections 21A are further turned back to adhere to the hook-and-loop fastener loop section 21B), whereby it is possible to improve stability of the position of the brain wave measurement device 1 at the time of wearing.

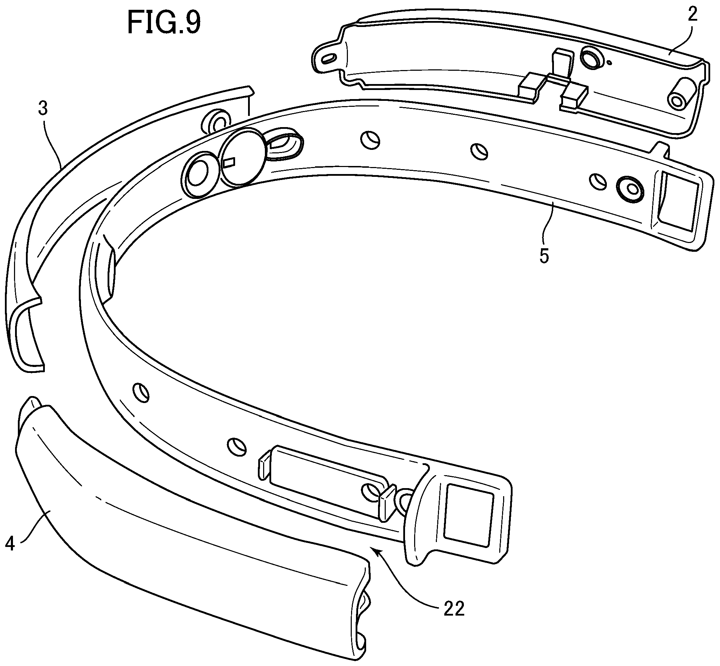

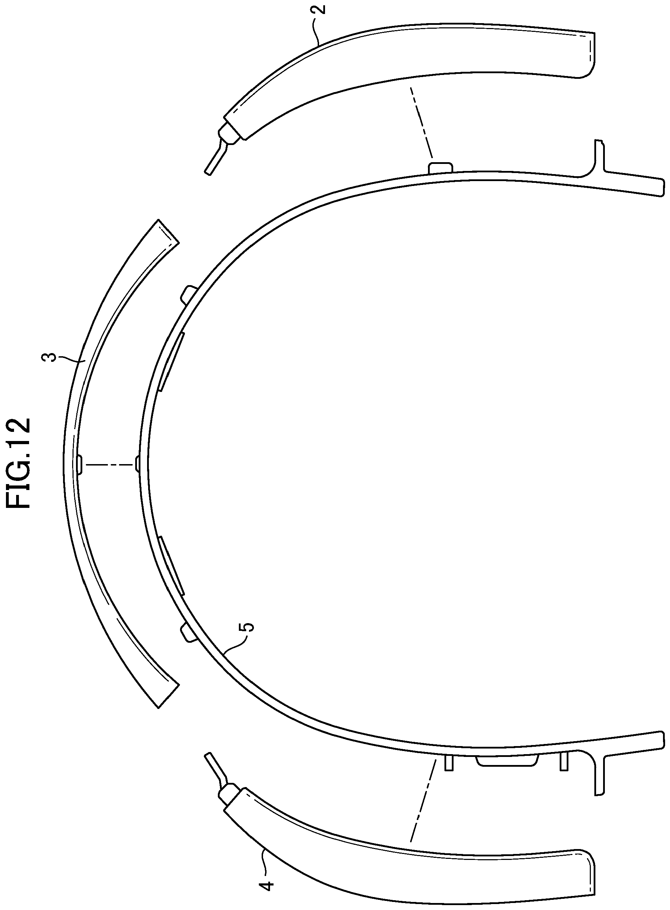

Figure 9 to Figure 12 are exploded views (Figure 9 to Figure 11 are perspective views and Figure 12 is a view from the upward direction of the user wearing the brain wave measurement device) at the time when the housing unit is disassembled into the constituent components. Wearing feeling of the user is improved by forming the front-side components in a divided structure. In one example, the signal processing unit 25 and a communication unit 29 shown in Figure 13 are configured by disposing circuit elements, elements, and the like on a circuit board. The circuit board is disposed in a circuit board housing position 22 in a space between the left front-side component 4 and the rear-side component 5 (the position of the circuit board is optional and the circuit board is preferably disposed between the left front-side component 4 and the rear-side component 5 or between the right front-side component 2 and the rear-side component 5. In a form in which the rigidity of the left front-side component 4 and the rigidity of the right front-side component 2 are set higher than the rigidity of the central front-side component 3 and higher than the rigidity of the rear-side component 5, the circuit board is protected from shocks by adopting such disposition of the circuit board). The right front-side component 2, the central front-side component 3, the left front-side component 4, and the rear-side component 5 are manufactured by selecting materials, shapes, and the like such that rigidities of at least two components among those components are different from each other. In particular, it is preferable to manufacture the components such that the rigidity of the right front-side component 2 and the rigidity of the left front-side component 4 are higher than the rigidity of the central front-side component 3 and higher than the rigidity of the rear-side component 5. A reinforcing member can be used for screwing the central front-side component 3 and the rear-side component 5.

Note that "rigidity " in this embodiment is decided according to, in the case of a member having fixed length, a Young's modulus (a modulus of longitudinal elasticity) of a material and a sectional secondary moment due to a sectional shape. That is, under the premise that the lengths of components are the same, in the case of components having the same sectional shape, when a Young's modulus of a material of a certain component is higher than a Young's modulus of a material of another component or, in the case of components of materials having the same Young's modulus, when a sectional secondary moment of a certain component is larger than a sectional secondary moment of another component, "the rigidity of the certain component is higher than the rigidity of the other component". However, in this embodiment, contribution of the Young's modulus due to the material to the rigidity of the component is set larger than contribution of the sectional secondary moment due to the sectional shape. That is, more appropriate rigidity of each component is attained by mainly appropriately selecting a material for each component. As a measurement method for a Young's modulus, for example, the right front-side component 2 is punched to manufacture a sample, the longitudinal and lateral widths of which in a surface direction (a direction of a surface generally parallel to the rear-side component 5 at the time of housing unit formation) is approximately 1.8 mm and the thickness of which in a direction perpendicular to the surface direction (which is strictly a curved surface but is approximately regarded as a plane) is approximately 0.1 mm, and the sample is used as a test sample, a tensile test is carried out in a 20°C physiological saline solution using Shimadzu Precision Universal Testing Machine Autograph AG-IS MS type manufactured by Shimadzu Corporation as described in the paragraph [0118] of the specification of Japanese Patent No. 6857784, and a Young's modulus (MPa) is calculated (tensile speed is 100 mm/minute) as a tensile modulus of elasticity from a stress-elongation curve, whereby a Young's modulus of the right front-side component 2 can be measured. Young's moduli of the other components such as the central front-side component 3, the left front-side component 4, and the rear-side component 5 can be measured in the same manner ("a surface direction" in a sample of the rear-side component 5 may be, for example, a direction of a surface generally parallel to "a surface direction" of a sample of the right front-side component 2). The materials of the right front-side component 2, the central front-side component 3, the left front-side component 4, and the rear-side component 5 may be optional. Numerical values of Young's moduli of those components may also be any values. However, in one example, the components can be manufactured as follows (since physical property values of the materials are specification values published by manufacturers, test methods are different from one another but magnitude relations among Young's moduli do not change even if the test methods are unified):
a Young's modulus (a tensile modulus of elasticity) of the central front-side component 3 is 49.5 MPa (megapascal) (a material manufactured by Du Pont-Toray Co., Ltd.: thermoplastic polyester elastomer Hytrel (R), grade: 4047N. A test method conforms to JIS K7113-1995);
both of Young's moduli (tensile moduli of elasticity) of the right front-side component 2 and the left front-side component 4 are 2550 MPa (megapascal) (a material manufactured by Mitsubishi Engineering Corporation: PBT resin (polybutylene terephthalate resin) Novaduran (R), grade: 5010R5. A test method conforms to ISO 527-1 and 527-2); and
a Young's modulus of the rear-side component (a tensile modulus of elasticity) is 1350 MPa (megapascal) (a material manufactured by Japan Polypropylene Corporation: PP (polypropylene) Novatec (R), grade: BC4BSW. A test method conforms to JIS K7161 7162: 1994).

A sectional secondary moment can be calculated from a sectional shape by a publicly-known formula. By reducing the thickness in the normal direction of the central front-side component 3 located substantially in the center of the housing unit, it is possible to form the sectional shape of the central front-side component 3 to have a smaller sectional secondary moment and further reduce the rigidity of the central front-side component 3.

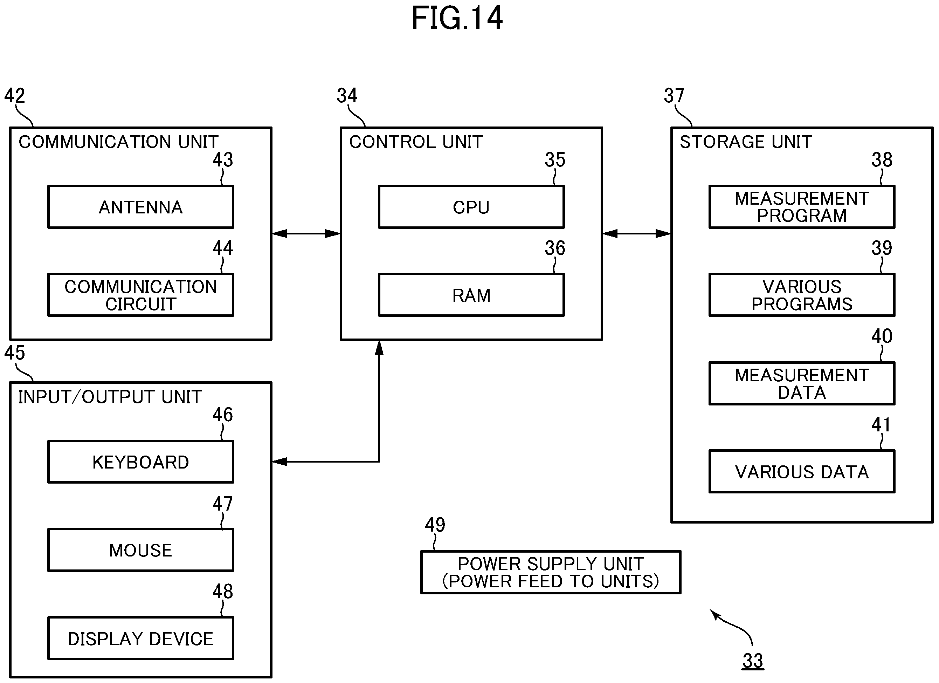

Figure 13 is a block diagram showing a configuration of the brain wave measurement device according to the embodiment of the present invention. Figure 14 is a block diagram showing a configuration of a data collection terminal device. In this embodiment, brain wave data obtained in measurement by the brain wave measurement device 1 is transmitted from the brain wave measurement device 1 to a data collection terminal device 33 and analysis processing and the like for the brain wave data is performed in the data collection terminal device 33.

The brain wave measurement device 1 shown in Figure 13 includes a measurement electrode 6 to a measurement electrode 23 (if one measurement electrode is provided, the measurement electrode 23 is unnecessary), which are N (N is a natural number equal to or larger than one) measurement electrodes, the REF electrode (reference electrode) 8, the GND electrode (ground electrode) 24, the signal processing unit 25, the communication unit 29, the operation unit 10, the display LED 11, and the power supply unit 32. As described above, the electrodes are separately connected to the signal processing unit 25. Electric signals from the electrodes are input to an amplification circuit 26 of the signal processing unit 25.

The signal processing unit 25 includes the amplification circuit 26, an A/D converter (analog-to-digital converter) 27, and a digital signal processing unit 28. The amplification circuit 26 is a circuit that amplifies living body potential input from various electrodes as electric signals. The amplification circuit 26 performs processing for, for example, measuring a potential difference between the measurement electrode 6 and the reference electrode 8 and amplifying this potential difference and then outputting the potential difference to the A/D converter 27 and measuring a potential difference between the measurement electrode 7 and the reference electrode 8 and amplifying this potential difference and then outputting the potential difference to the A/D converter 27 (the same applies when the number of measurement electrodes is three or more). The A/D converter 27 is a conversion circuit that converts an analog signal into a digital signal. The A/D converter 27 converts the various potential differences described above input from the amplification circuit 26 as analog signals from the analog signals into digital signals and outputs the digital signals to the digital signal processing unit 28. In one example, the digital signal processing unit 28 is configured from a CPU, a memory device such as a RAM (random access memory) or a ROM (read only memory), and the like. The digital signal processing unit 28 processes the digital signals input from the A/D converter 27 and, for example, generates a digital signal indicating a potential difference between the measurement electrode 6 and the reference electrode 8 as a numerical value and generates a digital signal indicating a potential difference between the measurement electrode 7 and the reference electrode 8 as a numerical value (the same applies when the number of measurement electrodes is three or more) and outputs the digital signals to a communication circuit 31 of the communication unit 29. The CPU executes a program stored in the memory device, whereby the digital signal processing unit 28 may perform processing for, for example, executing FFT (fast fourier transformation) on the digital signals input from the A/D converter 27 and output a digital signal indicating an obtained result to the communication circuit 31 of the communication unit 29.

The communication unit 29 includes an antenna 30 and the communication circuit 31. The communication circuit 31 transmits the digital signal input from the digital signal processing unit 28 to the data collection terminal device 33 via the antenna 30. In one example, the communication unit 29 wirelessly communicates with a communication unit 42 of the data collection terminal device 33 with a BLE (bluetooth low energy) method.

The operation unit 10 is the power button 10 as described above. The user presses the power button 10, whereby ON (the operation state) and OFF (the stop state) of the operation of the brain wave measurement device 1 are switched. Lighting, extinction, flashing, and a light emission color of the display LED 11 are switched according to the operation state and the charging state. The power supply unit 32 includes a lithium ion battery, a circuit for supplying electric power to the units of the brain wave measurement device 1, and the like, and is disposed in the housing unit.

The data collection terminal device 33 shown in Figure 14 includes a control unit 34, a storage unit 37, the communication unit 42, an input/output unit 45, and a power supply unit 49.

The control unit 34 includes a CPU 35 and includes a RAM 36 as a temporary memory. The CPU 35 executes a measurement program 38 recorded in the storage unit 37, whereby the CPU 35 processes brain wave measurement data received from the brain wave measurement device 1 and performs various kinds of measurement processing (when the FFT described above is performed on the data collection terminal device 33 side, a program for executing the FFT is stored in the storage unit 37 as the measurement program 38). The CPU 35 executes various programs 39 such as an OS (operating system) and various applications stored in the storage unit 37 to execute and control various operations of the data collection terminal device 33.

The storage unit 37 is a recording device including a hard disk drive, an SSD (solid state drive), or the like, and stores the measurement program 38 and the various programs 39 described above. The storage unit 37 stores measurement data 40 (for example, data of an analysis result obtained by executing FFT processing) and various data 41.

The communication unit 42 includes an antenna 43 and a communication circuit 44. The communication circuit 44 performs, via the antenna 43, data transmission and reception such as reception of brain wave measurement data from the brain wave measurement device 1. In one example, the communication unit 42 wirelessly communicates with the communication unit 29 of the brain wave measurement device 1 with the BLE method.

The input/output unit 45 includes a keyboard 46 for an operator of the data collection terminal device 33 (a person who performs an analysis of brain wave measurement data) to input instructions and data to the data collection terminal device 33, a mouse 47, and a display device 48 (a liquid crystal display device, an organic electroluminescence (organic EL) display device or the like) for performing various kinds of display. Besides, the input/output unit 45 may include an output device such as a speaker.

The power supply unit 49 includes a circuit for receiving power feed from an external power supply and supplying electric power to the units of the data collection terminal device 33, and the like, and may include a battery such as a lithium ion battery.

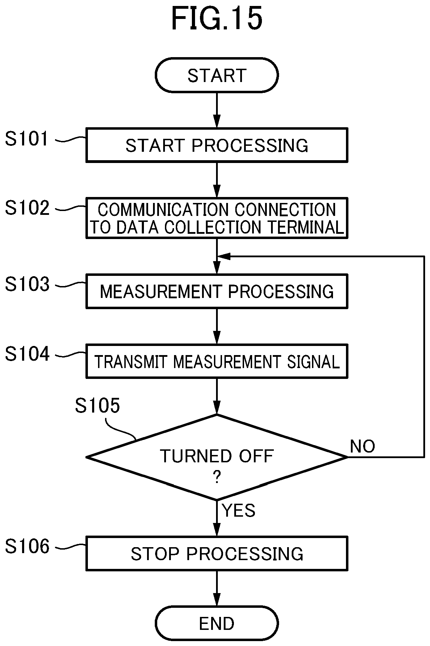

Figure 15 is a flowchart showing operations of the brain wave measurement device and the data collection terminal device according to the embodiment of the present invention. First, the user of the brain wave measurement device 1 (a person to be tested) continuously presses the power button 10 for approximately one to two seconds to thereby cause the brain wave measurement device 1 to start (step S101). Note that it is assumed that the data collection terminal device 33 has already started. When the brain wave measurement device 1 starts, on condition that BLE connection is enabled on the data collection terminal device 33 side, BLE connection is established between the communication unit 29 of the brain wave measurement device 1 and the communication unit 42 of the data collection terminal device 33 (step S102). The user of the brain wave measurement device 1 wears the brain wave measurement device 1 on the head of the user as shown in Figure 7, brings the measurement electrodes 6 and 7 into contact with the frontal region of the user such that, preferably, the positions of the measurement electrodes 6 and 7 become symmetrical to each other from the center line of the head and brings the reference electrode 8 into contact with the ear of the user. When the ground electrode 24 is included in the brain wave measurement device 1, the user brings the ground electrode 24 into contact with the head of the user or any position of the body of the user.

In this state, the potential difference between the potential of the measurement electrode 6 and the potential of the reference electrode 8 is amplified by the amplification circuit 26, an amplified analog signal is converted into a digital signal by the A/D converter 27, the digital signal generated by the conversion by the A/D converter 27 is processed by the digital signal processing unit 28 (step S103), and a digital signal generated by the processing and indicating a temporal change in the potential difference between the potential of the measurement electrode 6 and the potential of the reference electrode 8 is transmitted from the communication unit 29 of the brain wave measurement device 1 to the communication unit 42 of the data collection terminal device 33 (step S104). Similarly, the potential difference between the potential of the measurement electrode 7 and the potential of the reference electrode 8 is amplified by the amplification circuit 26, an amplified analog signal is converted into a digital signal by the A/D converter 27, the digital signal generated by the conversion by the A/D converter 27 is processed by the digital signal processing unit 28 (step S103), and a digital signal generated by the processing and indicating a temporal change in the potential difference between the potential of the measurement electrode 7 and the potential of the reference electrode 8 is transmitted from the communication unit 29 of the brain wave measurement device 1 to the communication unit 42 of the data collection terminal device 33 (step S104). When three or more measurement electrodes are provided, similarly, digital signals indicating temporal changes in potential differences between the potentials of the measurement electrodes and the potential of the reference electrode 8 are generated and transmitted from the communication unit 29 of the brain wave measurement device 1 to the communication unit 42 of the data collection terminal device 33. These kinds of processing on the brain wave measurement device 1 side are continuously repeatedly performed at a predetermined time interval unless the brain wave measurement device 1 is turned off by the power button 10 of the brain wave measurement device 1 being continuously pressed again for approximately one to two seconds (NO in determination processing in step S105). When execution of a measurement application (included in the measurement program 38) is started by the CPU 35 of the data collection terminal device 33 according to input of the operator of the data collection terminal device 33, the CPU 35 executing the measurement program 38 continuously causes, based on a digital signal received from the brain wave measurement device 1, the storage unit 37 to continuously store, as the measurement data 40, brain wave data (for example, temporal change data of a potential difference) of respective channels (in one example, the potential difference between the potential of the measurement electrode 7 and the potential of the reference electrode 8 is set as a potential difference of the channel 1 and the potential difference between the potential of the measurement electrode 6 and the potential of the reference electrode 8 is set as a potential difference of a channel 2). The storage of the brain wave data in the storage unit 37 is ended according to input (tap on a measurement end button on the display device 48) of the operator of the data collection terminal device 33. The BLE connection between the brain wave measurement device 1 and the data collection terminal device 33 is released (cut) according to input (release of the communication connection to the brain wave measurement device 1) of the operator of the data collection terminal device 33. When the brain wave measurement device 1 is turned off by the power button 10 of the brain wave measurement device 1 being continuously pressed for approximately one to two seconds again (YES in the determination processing in step S105), the operation of the brain wave measurement device 1 stops (step S106).

### Example

A brain wave measurement device having a configuration described below was manufactured as an example of the brain wave measurement device of the present invention and a performance test was performed.
(The brain wave measurement device in the example)
Shape: the shape shown in Figures 9 to 12
Number of measurement electrodes (forehead electrodes): two (CH1, CH2)
Interval (between contact surface centers) of the forehead electrodes: 60 mm
Shape of the forehead electrodes: a plane type, a contour of a contact surface has a circular shape having a diameter of 15 mm
Shape of a reference electrode (an ear electrode): a convex type, a contour of a contact surface has a circular shape having a diameter of 15 mm
Materials of the constituent components of the housing unit
(The central front-side component 3) Thermoplastic polyester elastomer Hytrel (R) manufactured by Du Pont-Toray Co., Ltd., grade: 4047N. A Young's modulus 49.5 MPa (A test method conforms to JIS K7113-1995)
(The right front-side component 2 and the left front-side component 4) PBT resin (polybutylene terephthalate resin) Novaduran (R) manufactured by Mitsubishi Engineering Corporation, grade: 5010R5. A Young's modulus 2550 MPa (A test method conforms to ISO 527-1 and 527-2)
(The rear-side component) A material manufactured by Japan Polypropylene Corporation: PP (polypropylene) Novatec (R), grade: BC4BSW. A Young's modulus 1350 MPa (A test method conforms to JIS K7161 7162: 1994)

When a subject wore the brain wave measurement device having the configuration described above and checked wearing feeling, wearing feeling around the forehead electrodes was improved compared with trial products of a convex curved electrode (a diameter 15 mm) and a concave curved electrode (a diameter 20 mm). Pinching strength of the ear electrode was improved compared with a flat trial product having a diameter of 11 mm. Tightened feeling around the temples was reduced and pain was eliminated by selecting the materials described above.

Further, a brain wave measurement test of the subject was performed using the brain wave measurement device having the configuration in the example described above and consistency of a result with a brain wave measurement test performed using Polymate (R) (Miyuki Giken Co., Ltd.), which is an existing measurement device, was verified. Correlation coefficients of both the test results are shown in Table 1 to Table 4 below.

**[Table 1]**

| CH | CH1:left | | | | | |
|---|---|---|---|---|---|---|
| Eye state | Eye Opened (Eye Opened) | | | | | |
| Signal | raw (raw) | delta (delta) | theta (theta) | alpha (alpha) | beta (beta) | gamma (gamma) |
| Subject 1 | 0.92 | 0.98 | 0.98 | 0.91 | 0.85 | 0.83 |
| Subject 2 | 0.95 | 0.99 | 1 | 0.96 | 0.83 | 0.46 |

**[Table 2]**

| CH | CH Cleft | | | | | |
|---|---|---|---|---|---|---|
| Eye state | Eye Closed (Eye Closed) | | | | | |
| Signal | raw (raw) | delta (delta) | theta (theta) | alpha (alpha) | beta (beta) | gamma (gamma) |
| Subject 1 | 0.75 | 0.8 | 0.87 | 0.85 | 0.83 | 0.88 |
| Subject 2 | 0.88 | 0.85 | 0.9 | 0.97 | 0.83 | 0.74 |

**[Table 3]**

| CH | CH2: right | | | | | |
|---|---|---|---|---|---|---|
| Eye state | Eye Opened (Eye Opened) | | | | | |
| Signal | raw (raw) | delta (delta) | theta (theta) | alpha (alpha) | beta (beta) | gamma (gamma) |
| Subject 1 | 0.9 | 0.98 | 0.98 | 0.88 | 0.92 | 0.91 |
| Subject 2 | 0.95 | 0.99 | 1 | 0.97 | 0.64 | 0.24 |

**[Table 4]**

| CH | CH2: right | | | | | |
|---|---|---|---|---|---|---|
| Eye state | Eye Closed (Eye Closed) | | | | | |
| Signal | raw (raw) | delta (delta) | theta (theta) | alpha (alpha) | beta (beta) | gamma (gamma) |
| Subject 1 | 0.56 | 0.39 | 0.72 | 0.82 | 0.8 | 0.85 |
| Subject 2 | 0.84 | 0.86 | 0.85 | 0.97 | 0.72 | 0.46 |

As described above, in the brain wave measurement device in the example, satisfactory results were obtained in both of the wearing feeling of the subject and the consistency of the measurement results with the existing brain wave measurement device.

### Industrial Applicability

The present invention is usable for brain wave measurement in any industries including medical equipment and research equipment industries.

### Reference Signs List

- 1: Brain wave measurement device
- 2: Right front-side component
- 3: Central front-side component
- 4: Left front-side component
- 5: Rear-side component
- 6: (Right-side) measurement electrode (forehead electrode)
- 7: (Left-side) measurement electrode (forehead electrode)
- 8: Reference electrode (REF electrode, ear electrode)
- 9: (Coated) reference electrode lead wire
- 10: Power button (operation unit)
- 11: Display LED
- 12: Charging port
- 13: Lid of charging port
- 14: (Right-side) nonslip sheet
- 15: (Left-side) nonslip sheet
- 16: (Right-side) auxiliary band attachment hole
- 17: (Left-side) auxiliary band attachment hole
- 18: Human body head
- 19: Wearing auxiliary band
- 20: Ring-like member
- 21A: Hook-and-loop fastener (hook)
- 21B: Hook-and-loop fastener (loop)
- 22: Circuit board housing position
- 23: N-th measurement electrode (N is 2 or more)
- 24: Ground electrode (GND electrode)
- 25: Signal processing unit
- 26: Amplification circuit
- 27: A/D (analog/digital) converter
- 28: Digital signal processing unit
- 29: Communication unit
- 30: Antenna
- 31: Communication circuit
- 32: Power supply unit (lithium ion battery or the like)
- 33: Data collection terminal device
- 34: Control unit
- 35: CPU
- 36: RAM
- 37: Storage unit
- 38: Measurement program
- 39: Various programs
- 40: Measurement data
- 41: Various data
- 42: Communication unit
- 43: Antenna
- 44: Communication circuit
- 45: Input/output unit
- 46: Keyboard
- 47: Mouse
- 48: Display device
- 49: Power supply unit

## Claims

1. A brain wave measurement device wearable on a living body head, the brain wave measurement device comprising:
a housing unit including at least a central front-side component, a left front-side component, a right front-side component, and a rear-side component, the housing unit having a curved shape such that the housing unit is disposed along the living body head from a left temporal region through a frontal region to a right temporal region at a time of wearing;
at least one measurement electrode that is fixed to the rear-side component and comes into contact with the frontal region at the time of wearing; and
a signal processing unit housed in the housing unit, the signal processing unit processing an electric signal obtained via the measurement electrode, wherein
rigidities of at least two components among the central front-side component, the left front-side component, the right front-side component, and the rear-side component are different from each other.

2. The brain wave measurement device according to claim 1, wherein a number of the measurement electrodes is at least two or more.

3. The brain wave measurement device according to claim 1 or 2, wherein rigidities of the left front-side component and the right front-side component are higher than rigidity of the central front-side component.

4. The brain wave measurement device according to claim 2 or 3, wherein centers of respective surfaces of the measurement electrodes coming into contact with the frontal region at the time of wearing are separated to left and right from each other by 40 mm or more and 90 mm or less along a shape of the rear-side component.

5. The brain wave measurement device according to any one of claims 1 to 4, wherein a contour of a surface of the measurement electrode coming into contact with the frontal region at the time of wearing has a circular shape having a diameter of 10 mm to 25 mm.

6. The brain wave measurement device according to any one of claims 1 to 5, wherein the signal processing unit is disposed between the left front-side component and the rear-side component or between the right front-side component and the rear-side component.
